# EUROPEAN PATENT SPECIFICATION

(11) **EP 1 106 985 B1**
(45) Date of publication and mention of the grant of the patent: **04.05.2011**
(21) Application number: 00126862.2
(22) Date of filing: 07.12.2000
(51) Int. Cl.: G01N 15/06

(54) **Method and apparatus for analyzing particulate matter in gas.**
Verfahren und Vorrichtung zur Analyse von Teilchenmaterial in Gas.
Procédé et dispositif d'analyse de matériau particulaire dans un gaz.

(30) Priority: 10.12.1999 JP 35106799; 29.03.2000 JP 2000092283; 30.08.2000 JP 2000261344
(43) Date of publication of application: 13.06.2001
(73) Proprietor: HORIBA, LTD., Kyoto (JP)
(72) Inventor: Uchihara, Hiroshi, Horiba, Ltd., Kyoto (JP); Okayama, Junji, Horiba, Ltd., Kyoto (JP); Bando, Atsushi, Horiba, Ltd., Kyoto (JP); Ikeda, Masahiko, Horiba, Ltd., Kyoto (JP); Adachi, Masayuki, Horiba, Ltd., Kyoto (JP); Asano, Ichiro, Horiba, Ltd., Kyoto (JP); Fukushima, Hirokazu, Horiba, Ltd., Kyoto (JP)
(74) Representative: Müller - Hoffmann & Partner

(56) References cited:
- EP-A- 0 269 511
- EP-A- 0 511 583
- US-A- 3 838 969
- US-A- 4 293 308
- US-A- 5 110 747
- US-A- 5 279 146

## Description

### Field of the Invention

This invention relates to a method for analyzing particulate matter (hereinafter to be referred to as PM) contained in a gas discharged for example from a diesel engine and an apparatus for performing said method.

### Description of the Prior Art

As a procedure for determining PM contained in an emission of a diesel engine, a filter weighing method for quantitative analysis is known in general which is based on the weight difference of the filter before and after capturing PM which comprises diluting a high temperature emission gas discharged from the diesel engine with clean air, capturing PM with a filter by inhaling a fixed capacity of the diluted emission, and weighing the filter with a precision balance or the like.

However, in the above filter weighing method, because of the large effect of the amount of water which is adsorbed to the filter on the measurement error, a constant temperature and constant moisture processing are required so as to keep the water content in the filter before and after the capture constant. Furthermore, when determining low concentrations of PM in the emission, it is necessary to accurately weigh amounts of PM in the range of 0.1 mg that are captured on a filter that weighs for example 200 mg. Thus, there is the problem that the measurement error of the weight of the filter itself gives a significant effect on the measurement error of the PM weight.

To overcome the above-mentioned difficulties, a procedure is suggested in USP 5,110,747, USP 5,196,170, USP 5,279,970, and USP 5,401,468 publications, wherein a filter which has captured PM is heated in a heating furnace by stepwise elevating the temperature, oxidizing the PM, and determining the PM with a gas analyzer.

However, the majority of PM is constituted of inorganic carbon called dry soot (hereinafter "dry soot"), hydrocarbons called SOF (soluble organic fraction, hereinafter "SOF"), and sulfuric acid hydrate called sulfate (hereinafter "sulfate"), and depending on the procedure described in the above publications, separation of high boiling SOF and reduction of sulfate in an oxidation atmosphere is difficult. Accordingly, it has turned out to be difficult to measure individually the concentration or the weight of the dry soot, SOF, and sulfate by separating them.

This invention has been made in consideration of the difficulties described above, and it is an object of the invention to provide a method and an apparatus for analyzing simply and in high precision the particulate matter in an engine emission by which dry soot, SOF, and sulfate in PM contained in an engine emission can be fractionated individually even in a trifle amount.

Further, elemental carbon, which is one of the carbon components contained in TSP (total suspended particulate matter) in atmospheric air, is involved in the oxidation of SO₂ into SO₄²-, and may cause climatic change depending on its concentration. One technique for analyzing the elemental carbon is to thermally separate elemental carbon and organic carbon, which is the other carbon component contained in TSP.

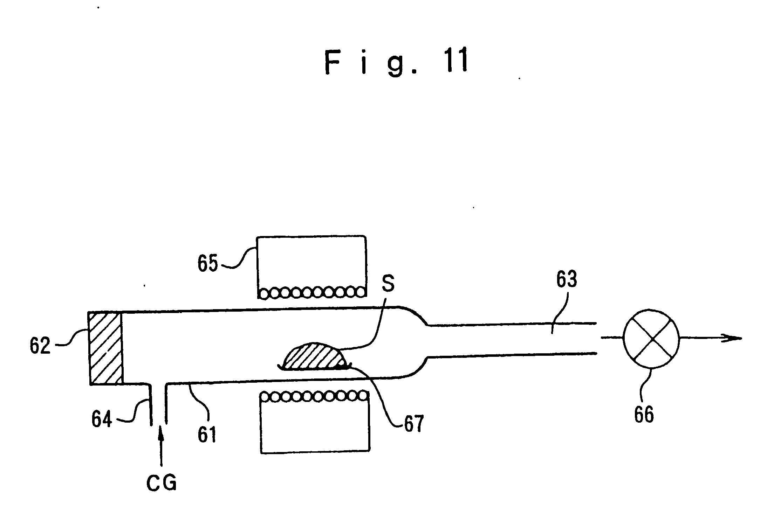

Fig. 11 is a schematic view showing a construction of a conventional carbon differentiating and analyzing apparatus. Referring to Fig. 11, a thermal decomposition tube 61 includes, on one end, a sample entrance 62 having a lid freely openable and closable for inserting a sample S, and on the other end, a gas exit 63. The thermal decomposition tube 61 further includes, on the sample entrance 62 side thereof (hereafter to be referred to as upstream side), an inlet 64 of carrier gas CG. A heater 65 is wound around the outer circumference of the thermal decomposition tube 61 on the downstream side of the carrier gas inlet 64, and the temperature of the heater 65 can be set to both a low temperature and a high temperature. A CO₂ analyzer 66 is connected to the gas exit 63 via a suitable passageway (not illustrated). Further, the sample S is mounted on a sample boat 67.

The operation of the carbon differentiating and analyzing apparatus having the aforesaid construction will be described. First, a sample boat 67 having a sample S mounted thereon is set at a predetermined position in the thermal decomposition tube 61. In this state, the heater 65 is set at a low temperature (for example, about 400 °C) to heat the sample S, whereby the organic carbon contained in the sample S is evaporated and converted to CO₂ which is then carried to the CO₂ analyzer 66 by the carrier gas CG to determine the CO₂ concentration.
Next, the heater 65 is set at a high temperature (for example, about 1000 °C) to further heat the sample S, whereby the elemental carbon contained in the sample S is thermally decomposed and converted to CO₂ and then carried to the CO₂ analyzer 66 by the carrier gas CG to determine the CO₂ concentration. Based on the CO₂ concentration obtained in each of the aforesaid measurements, the amount of the organic carbon and the amount of the elemental carbon in the sample S can be determined.

However, in the case of the aforesaid conventional carbon differentiating and analyzing apparatus, only a single heater 65 is used, so that it takes time to set the heater 65 from low temperature to high temperature, and takes more time to set the heater 65 from high temperature to low temperature. Moreover, since it is difficult to detect the HC component evaporating at low temperature with the use of the CO₂ analyzer, a HC gas sensor is needed. Further CO is generated, if the oxidation does not proceed sufficiently because of insufficient temperature and causes errors.

Document US 4,293,308 A discloses a method and an apparatus for analyzing the sulphur content in samples in order to quantitatively determine small percentages by means of sample combustion in a helium current enriched with oxygen, wherein subsequently catalytic oxidation and reduction of combustion gases and a gas chromatographic separation and detection in a thermal conductivity detector are performed. A provided sample and helium gas are enriched with oxygen and fed into a reactor, where at its inlet part a combustion is performed, followed by catalytic oxidation on the basis of an oxidizing layer of a tungsten trioxide.

Document US 3,838,969 A discloses a process of elemental analysis of sulphur containing materials, wherein in order to determine contents of carbon, hydrogen, nitrogen and sulphur by oxidation at elevated temperatures the carbon, hydrogen, nitrogen and sulphur are converted into carbon dioxide, water and oxides of nitrogen and oxides of sulphur, respectively.

Document US 5.110,747 A discloses a diesel particulate monitor, wherein diesel exhaust is measured by collecting the diesel particulate on a filter while measuring the amount of exhaust passing through the filter. The particulate material is heated in an oxygen-rich environment in order to oxidize carbon in order to form carbon dioxide. The amount of resulting carbon dioxide is measured in order to derive a corresponding value for the amount of filtered particulate.

Document EP 0 511 583 A2 discloses an apparatus for conditioning soot with the exhaust of an engine, wherein within a filter chamber having gas inlets and gas outlets a filter element is situated which is made of a metallic web. The filter element is operated in the form of an electrical driven resistive heating element. Supplies for air and/or inlet gas to the filter chamber are provided.

EP-A-0 269 511 refers to a method and an apparatus for determining at least two elements that are selected from hydrogen, carbon, sulfur and nitrogen that are included in at least two fractions of a sample of an organic material.

Furthermore, US 5,279,146 refers to a method and an apparatus for real-time measurement of particulate matter in combustion gases.

The present invention has been made in order to take the above-mentioned matters into account. It therefore is an object of the invention to provide a carbon differentiating and analyzing apparatus capable of differentiating and analyzing, with good precision, the organic carbon and the elemental carbon contained in a sample, which allows to perform a predetermined analysis with high precision in short time by reducing the period of time required for the analysis.

### SUMMARY OF THEINVENTION

The above objects are solved by the claimed matter according to the independent claims.

In order to attain the above object, the method for analyzing PM contained in gas comprises providing a filter which has caught PM contained in engine emissions in a heating furnace, firstly heating the filter to a predetermined temperature while feeding an inert gas into the heating furnace to evaporate SOF and sulfate contained in PM, oxidizing the evaporated SOF into CO₂, and reducing the evaporated sulfate into SO₂, analyzing the CO₂ and SO₂ with a gas analyzer unit, and thereafter, heating the filter while passing oxygen into the heating furnace to oxidize the PM remaining on said filter to convert it to CO₂, and analyzing the CO₂ with the gas analyzer unit.

And, as an apparatus for executing the above analyzing method, there is provided an apparatus comprising a gas feeder for selectively feeding an inert gas or oxygen to a heating furnace; a heating furnace for heating a filter which has captured the PM contained in the engine emission to a predetermined temperature while inert gas or oxygen is fed to the heating furnace; an oxidation reduction processor for oxidizing or reducing the gas generated during heating of the filter; and a gas analyzer unit for determining the concentrations of CO₂ and SO₂ in the gas supplied from the oxidation reduction processor.

According to the method for analyzing particulate matter in gas, a filter is installed in a flow passage in which an emission from an engine is passed, and an emission is passed in a predetermined flow amount and the PM in the emission is captured by the filter. This filter is provided in a heating furnace which is kept for example at 1000 °C, and firstly, the filter is heated while an inert gas such as nitrogen gas is fed into the heating furnace to gasify SOF and sulfate contained in PM, and the evaporated SOF is oxidized to convert it to CO₂, and the evaporated sulfate is reduced into SO₂. Then, the CO₂ and SO₂ are analyzed with a gas analyzer unit to obtain the CO₂ concentration and SO₂ concentration. These CO₂ concentration and SO₂ concentration are proportionate to the amounts of SOF and sulfate in PM, respectively. From these CO₂ concentration and SO₂ concentration and the total flow amount of the inert gas, the weights of CO₂ and SO₂ are obtained, and based on the weights of them, the weights of SOF and sulfate captured by the filter are obtained.

Subsequently, while feeding oxygen into the heating furnace, the filter is heated to oxidize the PM remaining on the filter (major part is dry soot) to generate CO₂, and said CO₂ is analyzed by the gas analyzer unit to obtain the CO₂ concentration. This CO₂ concentration is proportionate to the amount of dry soot in PM, and the weight of CO₂ is obtained from this CO₂ concentration and the total flow of oxygen. Based on this weight, the weight of the dry soot captured by the filter is obtained.

In the above method for analyzing PM contained in gas, during heating of the filter while feeding an inert gas into the heating furnace, firstly, low temperature heating is effected to an extent that the SOF of low boiling temperature should be evaporated, and thereafter, high temperature heating is effected to an extent that the SOF of high boiling temperature should be evaporated. When performing such method, differentiation can be made between the high boiling point SOF and the low boiling point SOF.

Further, in the aforesaid method of analyzing the PM contained in the gas, the aforesaid sulfate may be passed through heated quartz fibers to reduce the evaporated sulfate into SO₂. If this is carried out, the sulfate can be heated efficiently and with certainty, so that the sulfate can be reduced to SO₂ with more certainty.

A carbon differentiating and analyzing apparatus includes a thermal decomposition tube having a sample entrance formed at one end and a gas exit formed at the other end, and a CO₁ analyzer connected to the gas exit aide, wherein the thermal decomposition tube includes, successively from its upstream side, a carrier gas inlet, a low temperature heating part for evaporating organic carbon in the sample, and a high temperature heating part for thermally decomposing elemental carbon in the sample, where by the organic carbon is separated in the low temperature heating part and the elemental carbon is separated in the high temperature heating part

The aforesaid carbon differentiating and analyzing apparatus eliminates the need for the time required in raising the temperature and cooling in carbon differentiation and analysis.

Further, if a high temperature oxidizing part is provided midway between the high temperature heating part and the gas exit for oxidizing generated carbon compounds such as HC and CO into CO₂ HC and CO are prevented from flowing into the CO₂ analyzer.

Also, since a tube for introducing oxygen or air is connected at a site between the high temperature heating part and the high temperature oxidizing part of the thermal decomposing tube, low temperature CO in the presence of oxygen is completely converted into CO₂, whereby errors of the CO₂ analyzer caused by the generation of CO can be eliminated.

Furthermore, if a heating device is provided for heating the tube for introducing oxygen or air and a neighborhood of a site of connection between this tube and the thermal decomposition tube, the temperature of the gas generated by introduction of oxygen or air is prevented from decreasing, and the CO₂ concentration can be measured with a higher precision.

Still further, if a switching valve is provided at a site between the gas exit of the thermal decomposition tube and the CO₂ analyzer, gases that should not flow into the CO₂ analyzer, such as air flowing in from the outside and gas that is passed for purging the inside of the thermal decomposition tube, can be discharged to the outside.

### BRIRF DESCRIPTION OF THE DRAWINGS

FIG. 1 is a view to show schematically an example of the apparatus for analyzing the particulate matter in the gas according to this invention.
FIG. 2 is a view to show schematically an example of the sampling apparatus to capture PM on the filter.
FIG. 3 is a view for illustrating the motion of the invention.
FIG. 4 is a view to show schematically another example of the reduction processing part according to this invention.
FIG. 5 is a view to show schematically another example of the apparatus for analyzing the particulate matter in gas according to this invention.
Fig. 6 is a view schematically illustrating one example of a construction of a carbon differentiating and analyzing apparatus.
Fig. 7A is a graph schematically illustrating a temperature distribution in a thermal decomposition tube of the carbon differentiating and analyzing apparatus; and Fig. 7B is a graph schematically illustrating a result of analysis by the carbon differentiating and analyzing apparatus.
Fig. 8 is a view schematically illustrating another example of the carbon differentiating and analyzing apparatus.
Fig. 9 is a view schematically illustrating still an embodiment of the apparatus.
Fig. 10 is a view schematically illustrating still another embodiment of the apparatus.
Fig. 11 is a view schematically illustrating a construction of a conventional carbon differentiating and analyzing apparatus.

### DETAILED DESCRIPTION OF THE PREFERRED EMBODIMENTS

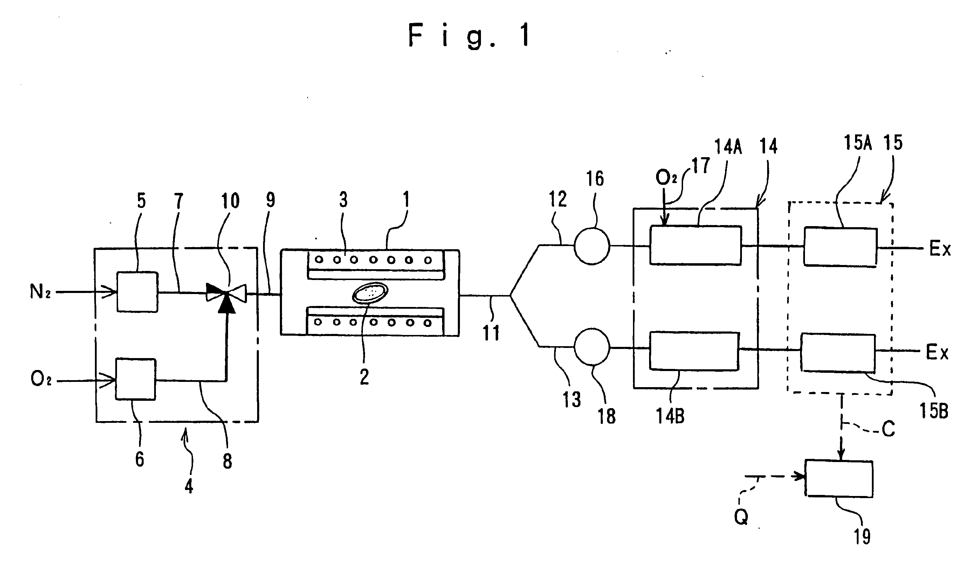

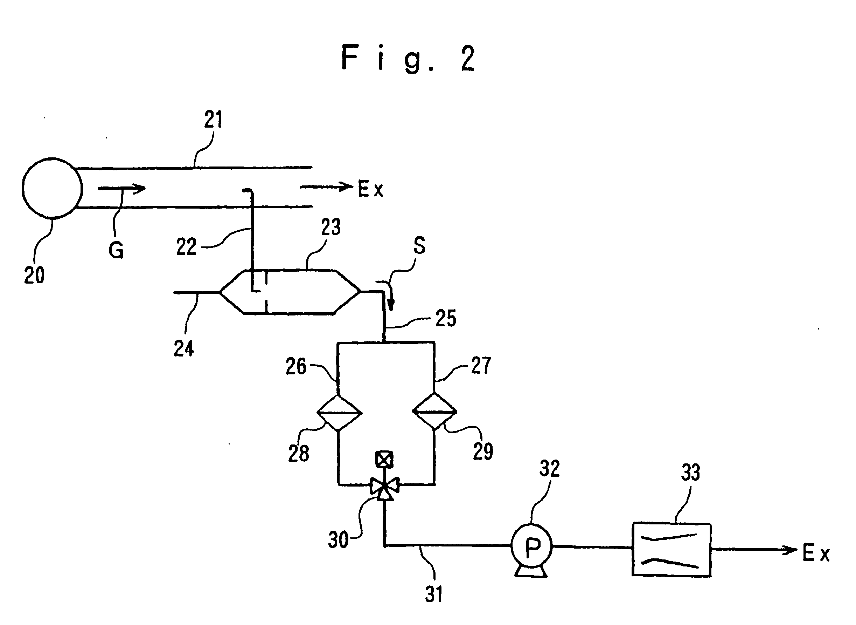

The invention is explained with reference to the drawings. FIG. 1 and FIG. 2 show a first embodiment of the invention. FIG. 1 schematically shows the design of an apparatus for analyzing the particulate matter in the gas. In the drawing, the numeral 1 depicts a heating furnace for heating a filter 2 (the apparatus for capturing PM will be described later) which has captured PM contained in the engine emission, comprising for example an electric furnace, which is equipped with a heater 3 for heating. The heating condition of the heater 3 is controlled by a temperature adjusting mechanism (not illustrated), and accordingly the temperature in the heating furnace 1 is set to the predetermined temperature.

The numeral 4 depicts a gas feeder for selectively feeding an inert gas (e. g. nitrogen gas) or oxygen to a heating furnace 1, which is constituted of a nitrogen gas feeding passage 7 and an oxygen feed passage 8 respectively furnished with flow controllers 5, 6 to determine and control the flow, and a three-way electromagnetic valve 10 connected to the downstream sides of these feed passages 7, 8 and the connection flow passage 9 to the heating furnace 1.

The numeral 11 depicts a gas flow passage through which the gas generated in the heating furnace 1 is passed. Its downstream side is branched into two parallel gas flow passages 12, 13. On the downstream sides of these gas flow passages 12, 13 are provided in series an oxidation reduction processor 14 and a gas analyzer unit 15, in this order.

More particularly, on one gas flow passage 12 there are provided, in this order, a flow meter 16 such as a mass flow meter, an oxygen feed passage 17 for feeding oxygen connected to an oxidation processor 14A, which contains an oxidation catalyst such as CuO, and a CO₂ analyzer 15A. On the other gas flow passage 13, there are provided a flow meter 18 similar to the above flow meter 16, a reducing processor 14B containing a reducing catalyst such as carbon, and an SO₂ analyzer 15B, in this order. The CO₂ analyzer 15A and SO₂ analyzer 15B to be used here are, for example, non-dispersion type infrared gas analyzers (NDIR gas analyzers).

In the oxidation reduction processor 14 the oxidation processor 14A and the reduction processor 14B are disposed in parallel with each other, and in the gas analyzer unit 15, the CO₂ analyzer 15A and the SO₂ analyzer 15B are disposed in parallel with each other.

The gas flow passages 9, 11 and the parts up to the gas analyzer unit 15 of the gas flow passages 12, 13 (including the oxidation processor 14A and reduction processor 14B) are heated and kept warm by a heating device (not illustrated) to keep a suitable temperature. This is in order to prevent a change in composition of the components contained in the gas formed in the heating furnace 1, and to insure oxidation and reduction processing.

Numeral 19 depicts an operation controller for performing operation based on the signals (flow signal Q, concentration signal C, etc.) from various parts of the apparatus or sending out control signals to various parts of the apparatus based on the results of the operation, comprising for example a personal computer.

The filter 2 is made for example from quartz having less impurities. In order to capture PM contained in engine emission on this filter 2, there is used for example a sampling apparatus which is constituted as shown in FIG. 2. The sampling apparatus is capable of passing a fixed flow amount of the emission from the engine. In FIG. 2, the numeral 20 depicts for example a diesel engine loaded on an automobile, and 21 depicts an exhaust pipe connected therewith. The part 22 is a probe inserted into the exhaust pipe 21 for sampling the emission G which flows in the exhaust pipe 21, and its downstream side is connected to the dilution tunnel 23 for diluting the sampled emission G.

A pipe 24 is connected to the upstream side of the dilution tunnel 23 for feeding the air for dilution.

The diluted sample gas S passes a gas flow passage 25 connected to the downstream side of the dilution tunnel 23. The dowstream side of this flow passage 25 is branched into two flow passages 26, 27. The respective flow passages 26, 27 are provided with filters 28, 29 for capturing PM contained in the sample gas S, so that one flow passage16 acts as a sample gas flow passage, and the other flow passage 27 acts as bypass flow passage for passing the emission when not sampling the PM, respectively. Of the filters 28, 29, one filter 28 is a filter for measurement, and the other filter 29 is a dummy filter.

Three-way electromagnetic valve 30 acts as flow passage changeover means and is provided on the downstream sides of the sample gas flow passage 26 and the bypass flow passage 27, and its downstream side is connected to the gas flow passage 31. This gas flow passage 31 is provided with a suction pump 32 whose suction capacity can be varied by controlling the number of revolutions, e. g., a Roots blower pump, and a flow meter 33 having high measurement precision, e. g., a venturimeter, in this order.

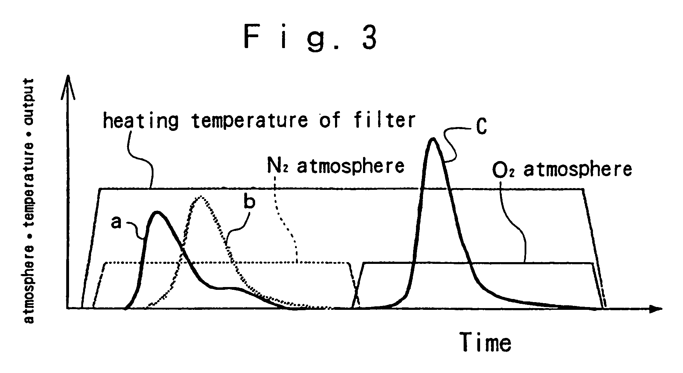

Next, a method for analyzing the particulate matter using the analyzer of the particulate matter in gas having the above constitution is explained with reference to FIG. 3. FIG. 3 shows in dependence on time the change of the atmosphere in the heating furnace 1, the heating temperature of the filter 2 and the output of CO₂ and SO₂. The marks a, c denote the CO₂ concentration outputs, and b shows the SO₂ concentration output.

At first, prior to the analysis, using a sampling apparatus shown in FIG. 2, emission G from the engine 20 is sampled by a fixed flow volume and PM in the emission G is captured on the filter 2.

Filter 2 which has captured PM is placed in the heating furnace 1 which is heated in advance to an inside temperature of 1000 °C. First, the electromagnetic valve 10 of the gas feeder 4 is actuated to connect the nitrogen gas feeding passage 7 with the heating furnace 1 to feed nitrogen gas into the heating furnace 1 through a flow controller 5. The flow amount of the nitrogen gas at this time is measured by the flow controller 5, and the results thereof are sent to the personal computer 19 as the flow signal Q.

In the above heating furnace 1, the filter 2 is heated to 1000 °C in a nitrogen gas atmosphere, by which the SOF and sulfate in the PM captured by the filter 2 are gasified, and they flow out into the gas flow passage 11 along with the nitrogen gas.

The gas sent out through the gas flow passage 11 (containing the gasified SOF and sulfate and other generated gases) flows into the gas flow passages 12, 13 and is divided into two parts. The gas which flows in gas flow passage 12 is fed into the oxidation processor 14A of the oxidation processor 14 through the flow meter 16. To this oxidation processor 14A oxygen is supplied, whereby the gasified SOF and the sulfate is oxidized by the oxidation catalyst and oxygen to be converted to CO₂ and H₂O, which, together with other gases, are sent to the CO₂ analyzer 15A of the gas analyzer unit 15 and their CO₂ concentration is measured. The gas which flows through the other gas flow passage 13 is fed to the reduction processor 14B of the oxidation reduction processor 14 through the flow meter 18, in which the sulfate out of the evaporated SOF and sulfate is reduced by the reducing catalyst to become SO₂, which is sent together with other gases to the SO₂ analyzer 15B of the gas analyzer unit 15 and the SO₂ concentration is determined

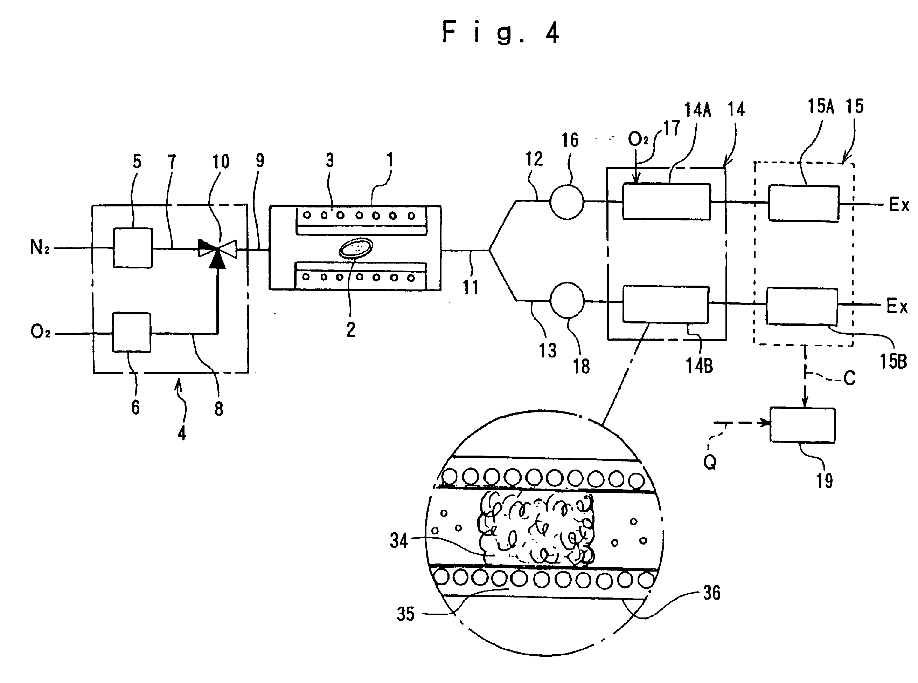

Here, referring to Fig. 4, reduction of sulfates into SO₂ may be promoted by disposing quartz fibers 34 instead of disposing a reduction catalyst in the aforesaid reduction processing part 148, and passing the sulfates through the quartz fibers 34.

In other words, SO₂, is obtained by evaporating the sulfates, and by heating the SO₃, the following reaction takes place:

SO₃ → SO₂ + (1/2)O₂ - 23.1 kcal.

In case the sulfates are not sufficiently heated by simply passing the sulfates through the inside of the tube 36 heated by the heater 35, the sulfates may be sent to the analyzer 15B without being reduced into SO₂. However, if the quartz fibers are disposed in the reduction processing part 14B as mentioned above, and heated by a heater in advance, and if the sulfates are passed through the heated quartz fibers, then the heat from the tube wall is efficiently transmitted to the sulfates by the quartz fibers when the sulfates pass through the quartz fibers, whereby the sulfates can be heated efficiently and with certainly, and the reduction to SO₂ can be carried out with more certainty. This makes it possible to measure the sulfates in the PM with good precision. Needless to say, it is possible to adopt a construction in which both the reduction catalyst and the quartz fibers are disposed in the aforesaid reduction processing part 148.

From the gas analyzer unit 15 there are outputted the CO₂ concentration and the SO₂ concentration signals in a form as shown for example by the marks a, b in FIG. 3, and they are inputted to the personal computer 19. As the signal to represent the flow of the nitrogen gas is inputted into the personal computer 19, based on these nitrogen gas flow, CO₂, concentration and SO₂ concentration, the weights of the SOF and sulfate in PM captured by the filter 2 are obtained respectively.

Next, the electromagnetic valve 10 of the above gas feeder 4 is changed over to connect the oxygen supply passage 8 with the heating furnace 1, and oxygen is fed into the heating furnace 1 through the flow controller 6. The oxygen flow at this time is measured by a flow controller 6, and the results thereof are sent to the personal computer 19 as flow signal.

In the above heating furnace 1, due to the heating of the filter 2 to 1000 °C in an oxygen atmosphere, the dry soot remaining on the filter 2 is burnt (oxidized) to generate CO₂ and CO (this is almost trifling in amount), which, together with oxygen, flow out to the gas flow passage 11.
The gases flowing out from the gas flow passage 11 (containing CO₂ and CO produced by the above combustion and other generated gases) are divided in the gas flow passages 12 and 13. The gas which flows through gas flow passage 12 is fed into the oxidation processor 14A of the oxidation reduction processor 14 through the flow meter 16. The oxidation processor 14A is fed with oxygen and CO which is contained in the above gas is oxidized to become CO₂. It is mixed with the CO₂ in the above gas and sent to the CO₂ analyzer 15A of the gas analyzer unit 15 together with other gases, when the CO₂ concentration is measured. The gas which flows through the gas flow passage 13 is fed into the reduction processor 14B of the oxidation reduction processor 14 through flow meter 18. In this case, the gas is sent to the SO₂ analyzer 15B of the gas analyzer unit 15 without undergoing any processing, and is wasted without having measurement of concentration.

From the above gas analyzer unit 15, a signal showing the CO₂ concentration in a form as shown in the mark c in FIG. 3 is outputted, which is inputted into the personal computer 19. As the signal representing the oxygen flow is inputted into the personal computer 19, based on these oxygen flow and CO₂ concentration, the weight of the dry soot in the PM captured by the filter 2 is obtained.

In this embodiment the emission gas G from the engine 20 is diluted during capturing PM by the filter. Therefore, by carrying out a calculation based on the weights of the dry soot, SOF and sulfate with the above dilution rate taken into account, the weights of the dry soot, SOF, and sulfate in the emission can be obtained.

As described above, according to the method and apparatus for analyzing particulate matter in gas of this invention, a filter 2 which has captured PM is accommodated in a heating furnace 1, and at first, the filter 2 is heated to a predetermined temperature in an inert gas atmosphere such as nitrogen gas to gasify SOF and sulfate. The gasified SOF and sulfate is further treated for oxidation or reduction to convert them into CO₂ and SO₂. Their concentrations are determined, and based on their concentrations the weights of SOF and sulfate in PM captured by the filter 2 are obtained. Then, the filter 2 is heated in the heating furnade 1 under oxygen atmosphere to generate CO₂, and based on said concentration the weight of the dry soot in the PM captured by the filter 2 is obtained. Accordingly, it is possible to fractionate individually the dry soot, SOF, and sulfate in the PM contained in the emission G from the engine 20 and measure them simply and in good precision.

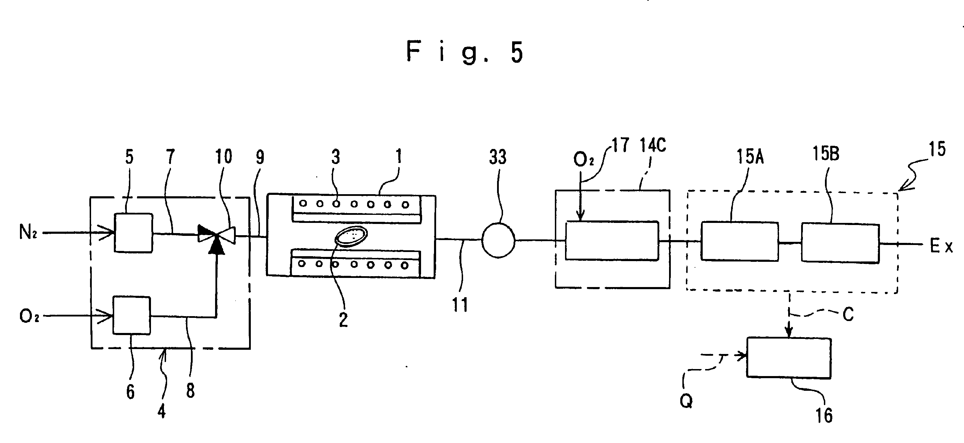

In the embodiment described above, the gas flow passage 11 provided on the downstream side of the heating furnace 1 is divided into two parts 12 and 13, and one gas flow passage 12 is provided with an oxidation processor 14A and a CO₂ analyzer 15A, and the other gas flow passage 13 is provided with a reduction processor 14B and a SO₂ analyzer 15B. As a result, the gas generated in the heating furnace 1 is divided into 1/2 part each, and inevitably the output signals obtainable in gas analysis becomes small. A constitution to dissolve this defect is explained with reference to FIG. 5, as the second embodiment.

In FIG. 5, the part 33 is a flow meter provided in the gas flow passage 11 on the downstream side of the heating furnace 1, being similar to the flow meters 16, 18 in the first embodiment. And, in the oxidation reduction processor 14C provided on the downstream side of the flow meter 33 an oxidation reduction catalyst having platinum (Pₜ) as principal component is provided, to which an oxygen supply passage 17 is connected. Further, in the gas analyzer unit 15 provided on the downstream side of the oxidation reduction processor 14C there are disposed in series a CO₂ analyzer 15A and an SO₂ analyzer 15B.

To explain the method for analyzing the particulate matter using an analyzing apparatus of the particulate matter in the gas shown in FIG. 5 above, firstly, prior to the analysis, using a sampling apparatus shown in FIG. 2, an emission G from the engine 20 is sampled by a predetermined flow and the PM in said emission G is captured in the filter 2.

The filter 2 which has captured PM is placed in the heating furnace 1 which is heated in advance to an inside temperature at 1000 °C. First, the electromagnetic valve 10 of the gas feeder 4 is actuated to connect the nitrogen gas feeding passage 7 with the heating furnace 1 to feed nitrogen gas into the heating furnace 1 through a flow controller 5. The flow amount of the nitrogen gas at this time is measured by the flow controller 5, and the results thereof are sent to the personal computer 19 as the flow signal.

In the above heating furnace 1, the filter 2 is heated to 1000 °C in a nitrogen gas atmosphere, by which the SOF and sulfate in the PM captured by the filter 2 are gasified, and they flow out into the gas flow passage 11 along with the nitrogen gas and other gases.

The gas sent out through the gas flow passage 11 (containing the gasified SOF and sulfate) is fed to the oxidation reduction processor 14 through flow meter 33. To this oxidation reduction processor 14C oxygen is supplied, wherein the SOF out of the gasified SOF and sulfate is oxidized by the oxidation reduction catalyst and oxygen and converted to CO₂ and H₂O, and on the other hand, the sulfate is reduced by the oxidation reduction catalyst and converted to SO₂. These CO₂, H₂O, and SO₂ are sent to the gas analyzer unit 15.

In the gas analyzer unit 15, at first the CO₂ concentration is measured in the CO₂ analyzer 15A, and then the SO₂ concentration is measured in the SO₂ analyzer 15B. From the gas analyzer unit 15 the CO₂ concentration and SO₂ concentration signals are outputted, and they are inputted to the personal computer 19. As the signal to represent the flow of the nitrogen gas is inputted into the personal computer 19, based on these nitrogen gas flow, CO₂ concentration and SO₂ concentration, the weights of the SOF and sulfate in PM captured by the filter 2 are obtained respectively.

Next, the electromagnetic valve 10 of the above gas feeder 4 is changed over to connect the oxygen supply passage 8 with the heating furnace 1, and oxygen is fed into the heating furnace 1 through the flow controller 6. The oxygen flow at this time is measured by a flow controller 6, and the results thereof are sent to the personal computer 19 as flow signal.

In the above heating furnace 1, due to the heating of the filter 2 to 1000 °C in an oxygen atmosphere, the dry soot remaining on the above filter 2 is burnt (oxidized) to generate CO₂ and CO (this is almost trifling in amount), which, together with oxygen, flow out to the gas flow passage 11.

The gases flowing out from the gas flow passage 11 (containing CO₂ and CO produced by the above combustion) are fed into the oxidation reduction processor 14 through the flow meter 33. As the oxidation reduction processor 14 is fed with oxygen, CO which is contained in the above gas is oxidized to become CO₂, and sent together with CO₂ in the above gas to the CO₂ analyzer 15A of the gas analyzer unit 15, where the CO₂ concentration is measured. This concentration signal is inputted to the personal computer 19. The gas which leaves the CO₂ analyzer 15A is sent to the SO₂ analyzer 15B, where it is wasted without undergoing measurement of concentration.

As the signal to represent the oxygen flow is inputted to the above personal computer 19, the weight of the dry soot in PM captured by the filter 2 is obtained based on the oxygen gas flow and the CO₂ concentration.

In this second embodiment as well, the emission G from the engine 20 is diluted in capturing PM by the filter 2, so that, in the same manner as in the first embodiment above, by making operation based on the weights of the dry soot, SOF and sulfate in consideration of the dilution rate, the weights of the dry soot, SOF and sulfate in the above emission G can be obtained.

As described above, according to the second embodiment as well, the dry soot, SOF and sulfate in PM contained in the emission G from the engine 20 can be individually fractionated and measured simply and in good precision.

In the second embodiment described above, the gas formed in the heating furnace 1 is not divided into two parts in the meantime, and in the gas analyzer unit 15, the gas is sequentially passed through the CO₂ analyzer 15A and the SO₂ analyzer 15B disposed in series, so that there can be obtained large concentration signals of CO₂ and SO₂. Also, this system has an advantage that the design of the apparatus is simple.

In the first and second embodiments described above, the filter 2 is first heated in the inert gas atmosphere in the heating furnace 1 to a predetermined temperature (1000 °C). Said heating may also be effected by a combination of low temperature heating and high temperature heating. This selection is explained as the third embodiment below.

The SOF contained in the emission G exhausted from the diesel engine 20 includes one originated from light oil and one originated from engine oil. The former SOF is evaporated at a relatively low temperature (e.g., about 350°C) and the latter SOF is evaporated at a relatively high temperature (about 700°C). In the first and second embodiments described above, the filter 2 is kept at a constant temperature when heated in the heating furnace 1 in an inert gas atmosphere, so that it is not possible to distinct between the SOF of light oil origin and the SOF of engine oil origin.

In the third embodiment, while heating the filter 2 under the inert gas atmosphere, the heating temperature of the filter 2 is first set to a low temperature so that the low boiling temperature SOF is evaporated, and thereafter to a high temperature so that the high boiling temperature SOF is evaporated. By carrying out the heating of the filter 2 under the inert gas atmosphere at two temperatures of low and high levels, it becomes possible to distinct the SOF of light oil origin and the SOF of engine oil origin.

To perform the heating procedure in two steps of low and high levels as described above, there are optional methods, for example, to change over the temperature in the heating furnace 1 by a temperature adjusting mechanism so as to heat the filter 2 first to a low temperature and then to heat the filter 2 to a high temperature. Alternatively, the heating furnace 1 can be provided inside with a low temperature heating unit and a high temperature heating unit, so as to make heating in the low temperature heating unit first, and to make heating by shifting the filter 2 to the high temperature heating unit.

This invention is not limited to the embodiments mentioned above but can be practiced by modification into various styles. For example, as a heating furnace 1, a high frequency heating furnace or an infrared image furnace may be used. And, as an inert gas to be supplied to the heating furnace 1, there may be used besides the nitrogen gas optional one such as argon gas.

During oxidation of the gasified SOF formed in heating the filter 2 at a predetermined temperature under the nitrogen gas atmosphere, in the oxidation processor 14A or the oxidation reduction processor 14 H₂O is formed along with CO₂. This H₂O may become an interference component in the measurement of CO₂ or GO₂. Accordingly, it may be eliminated before it is fed to the gas analyzer unit 15 or, as the CO₂ analyzer 15A or the SO₂ analyzer 15B to be provided in the gas analyzer unit 15, there may be used a gas analyzer of such constitution as can compensate the effect of the interference component.

Furthermore, as a gas analyzer unit to be provided in the gas analyzer unit unit 15, there may be provided a gas analyzer unit apparatus using a Fourier transform infrared spectrometer (FTIR gas analyzer unit apparatus). This FTIR gas analyzer unit apparatus is capable of measuring with one unit the concentrations of CO₂ and SO₂ simultaneously, and is also capable of measuring NOₓ and the like. Therefore, it is possible to measure at a stroke the concentrations of other components as well as the dry soot, SOF, and sulfate contained in PM. Furthermore, as a gas analyzer to be provided in the gas analyzer unit unit 15, a mass spectrometer (mass analyzer) may be used. This mass spectrometer is also capable of simultaneously measuring with a single unit the concentrations of CO₂, SO₂, NOₓ, and the like. Accordingly, when these FTIR gas analyzer apparatus or mass spectrometer are provided in the gas analyzer apparatus 15, the constitution of the whole apparatus is simplified.

As described above, according to this invention, it is possible to measure the dry soot, SOF and sulfate in the PM simply and in good precision. Especially, the dry soot, SOF and sulfate which occupy the major part in PM, which could hot be measured by the conventional filter weighting method or by the procedure disclosed in publication as listed in the beginning of this text, can be individually fractionated to measure the concentration or weight, and also the low concentration PM can be measured in good precision.

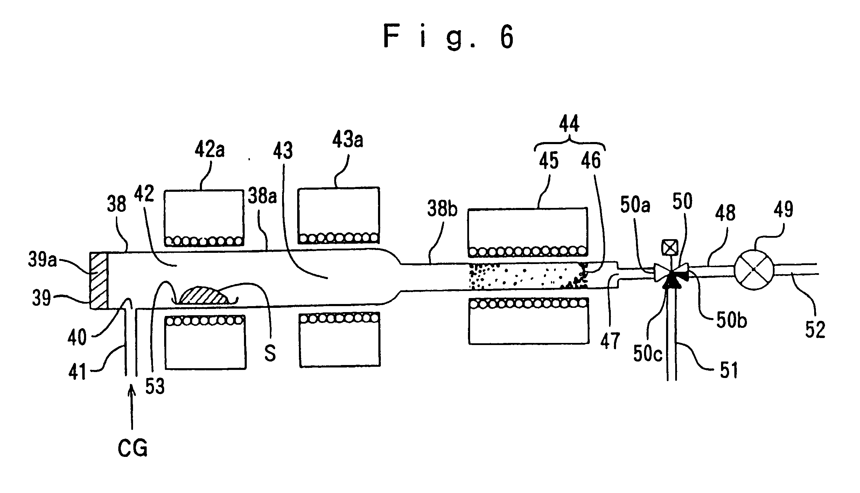

Hereafter, an example for further explaining the present invention will be described with reference to the attached drawings. Fig. 6 ia a view schematically illustrating one example of a construction of a carbon differentiating and analyzing apparatus of this example. Referring to Fig. 6, a thermal decomposition tube 38 includes, for example, a larger diameter part 38a and a small diameter part 38b. The thermal decomposition tube 38 is made of a haat-resistant material such as quartz or heat-resistant ceramics, and its heat-resisting temperature is above 1200°C. A sample entrance 39 is formed on one end (the large diameter part 38a side) of the thermal decomposition tube, and a freely openable and closable lid 39a is disposed there for inserting a sample S. A carrier gas inlet 40 is disposed in the neighborhood of the lid 39a, and a carrier gas passageway 41 is connected to the carrier gas inlet 40. A source of inert gas such as He or N₂ and a source of burning-aid gas such as O₂ or air (not illustrated) are disposed on the upstream side of the carrier gas passageway 41, whereby either one of the inert gas and the burning-aid gas can be selectively introduced as a carrier gas CG into the thermal decomposition tube 38.

A low temperature heating part 42 and a high temperature heating part 43 are disposed with a suitable spacing in the large diameter part 38a of the thermal decomposition tube. Heaters 42a, 43a both capable of setting an arbitrary temperature from room temperature to about 1200°C are wound, for example, around the large diameter part 38a, whereby organic carbon in the sample S is evaporated in the low temperature heating part 42, and elemental carbon in the sample S is thermally decomposed in the high temperature heating part 43.

A high temperature oxidizing part 44 is disposed in the small diameter part 38b connected to the aforesaid large diameter part 38a, and includes a heater 45 wound around the outer circumference of the small diameter part 38b and an oxidation catalyst 46 made of copper oxide, platinum, or the like formed in layers.

A gas exit 47 is formed on the downstream side of the small diameter part 38b, and a CO₂ analyzer 49 made, for example, of a non-dispersion type infrared gas analyzer (NDIR) is connected to the gas exit 47 via a passageway 48. A three-way solenoid valve 50 is a switching valve disposed midway in the passageway 48, and includes a first port 50a connected to the gas exit 47, a second port 50b connected to the CO₂ analyzer 49 side, and a third port 50c connected to a gas discharging passageway 51. During normal operation (while the power is off), the gas exit 47 side and the CO₂ analyzer 49 are in communication with each other. A gas discharging passageway 52 is disposed on the downstream side of the CO₂ analyzer 49.

A sample boat 53 is used to mount the sample S thereon, and is formed to have a size such that the sample boat 53 can be inserted into or taken out from the large diameter part 38a through the sample entrance 39, and the sample boat 53 can be slid freely in the large diameter part 38a.

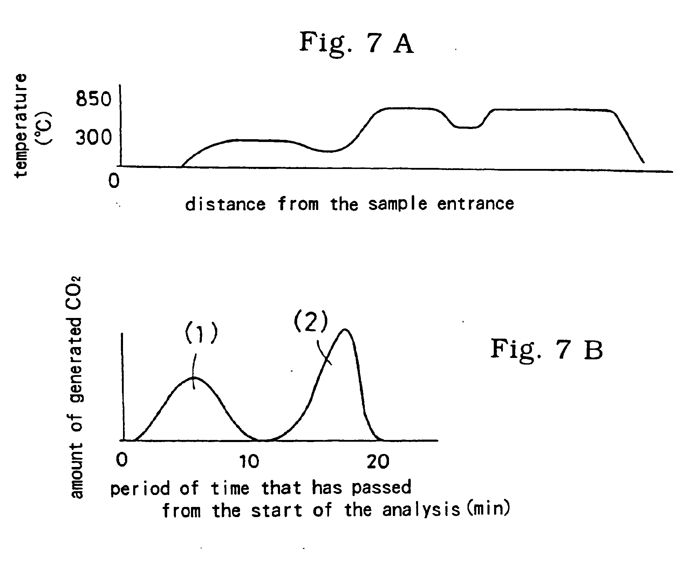

Next, the operation of the carbon differentiating and analyzing apparatus having the aforesaid construction will be described with reference also to Fig. 7. First, the lid 39a of the sample entrance 39 must be opened. At this time,in order to prevent air from flowing from the lid 39a side into the thermal decomposition tube 38 and to prevent CO₂ in the air from flowing to the CO₂ analyzer 49 side, the three-way solenoid valve 50 must be switched to allow the gas exit 47 and the gas discharging passageway 51 to be in communication with each other. Next, the sample S in a state of being mounted on the sample boat 53 is inserted into the thermal decomposition tube 38 so that the sample S will reach the low temperature heating part 42 which is kept at a temperature needed for evaporation of organic carbon, for example, about 500 °C under the He atmosphere and about 300°C under air atmosphere. Then, the three-way valve is switched to connect the gas exit 47 and the passageway 48 thereby to allow the CO₂ analyzer 49 and the gas exit 47 to be in communication with each other. In this state, the organic carbon is evaporated for example, for three to five minutes. Here, if the evaporation is to be carried out under He or N₂ atmosphere,

He or N₂ is introduced from the carrier gas inlet 40, and if the evaporation is to be carried out under air or O ₂ atmosphere, it is introduced also from the carrier gas inlet 40.

The organic carbon which has been evaporated by low temperature heating of the sample S in the low temperature heating part 42 is conveyed to the high temperature oxidizing part 44 by means of the carrier gas CG. Since the high temperature oxidizing part 44 is kept at a high temperature, for example, of 1000°C by the heater 45, HC and CO contained in the organic carbon is oxidized into CO₂. The resultant CO₂ gas is conveyed via the three-way solenoid valve 50 by the carrier gas CG to the CO₂ analyzer 49, where its concentration is measured.

After the evaporation of the organic carbon is carried out for a predetermined period of time, the sample S together with the sample boat 53 is moved to the high temperature heating part 43 which is kept at a temperature needed in thermal decomposition of elemental carbon (for example, about 850 °C). For this movement, the lid 39a of the sample entrance 39 must be opened. At this time, in order to prevent air from flowing from the lid 39a side into the thermal decomposition tube 38 and to prevent CO₂ in the air from flowing to the CO₂ analyzer 49 side, the three-way solenoid valve 50 must be switched to allow the gas exit 47 and the gas discharging passageway 51 to be in communication with each other.

Then, the sample S is heated at the aforesaid temperature for a predetermined period of time, for example, three to five minutes in the high temperature heating part 43 for thermal decomposition. The thermally decomposed elemental carbon is conveyed to the CO₂ analyzer 49 via the high temperature oxidizing part 44 by means of the carrier gas CG, in the same manner as the organic carbon. After the CO₂ concentration is measured in the CO₂ analyzer 49, the gas is discharged to the gas discharging passageway 52.

Fig. 7A is a graph schematically illustrating a temperature distribution in the thermal decomposition tube 38 of the carbon differentiating and analyzing apparatus. In the graph, the horizontal axis represents the distance from the sample entrance 39, and the vertical axis represents the temperature in the thermal decomposition tube 38.

Fig. 7B is a graph schematically illustrating the amount of generated CO₂, which is obtained by the aforesaid carbon differentiating and analyzing apparatus. In the graph, the horizontal axis represents the period of time that has passed from the start of the analysis, and the vertical axis represents the amount of generated CO₂. The first peak (1) in the graph indicates the amount of CO₂ generated by evaporation of the organic carbon, and the next peak (2) indicates the amount of CO₂ generated by thermal decomposition of the elemental carbon. Thus, the amounts of generated CO₂ respectively corresponding to the organic carbon and the elemental carbon are obtained. From these results, the amounts of the organic carbon and the elemental carbon contained in the sample S can be differentiated and analyzed.

As described above, in the carbon differentiating and analyzing apparatus of this example, the carrier gas inlet 40, the low temperature heating part 42 for evaporating the organic carbon in the sample S, and the high temperature heating part 43 for thermally decomposing the elemental carbon in the sample 3 are disposed in this order in the single thermal decomposition tube 38. Therefore, the low temperature heating part 42 and the high temperature heating part 43 can be simultaneously operated and each can be kept at a respective predetermined temperature. This eliminates the need for the time required in raising the temperature or cooling in the carbon differentiation and analysis, thereby all the more reducing the time needed for the analysis.

Further, the high temperature oxidizing part 44 for oxidizing the generated carbon compounds such as HC and CO into CO₂ is disposed on the downstream side of the high temperature heating part 43 of the thermal decomposition tube 38. Therefore, even if carbon compounds such as HC and CO are mixed in the gas generated when the sample S is heated at a low temperature in the low temperature heating part 42 and at a high temperature in the high temperature heating part 43, these carbon compounds are oxidized with certainty in the high temperature oxidizing part 44 into CO₂, thereby preventing errors from being generated in the measurement performed by the CO₂ analyzer 49.

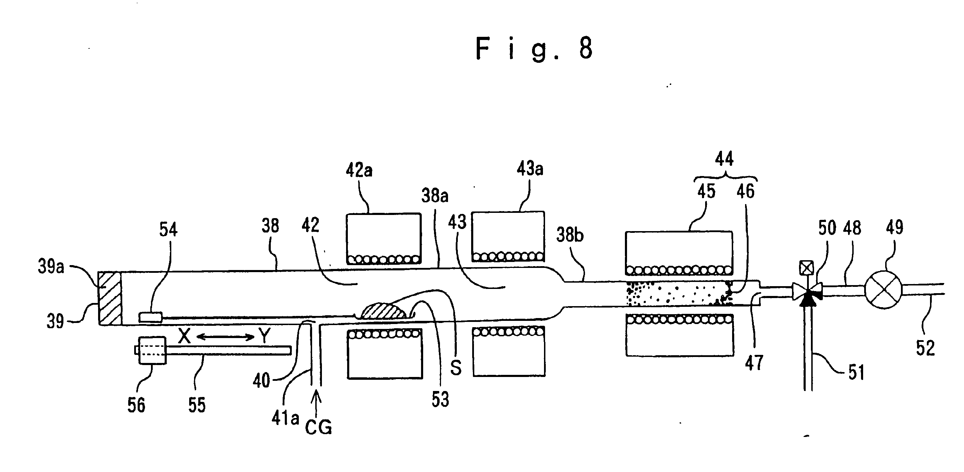

The carbon differentiating and analyzing apparatus can be varied with various different modifications. For example, it is possible to adopt a construction such that, as shown in Fig. 8, an iron supporting tool 54 is connected to the sample boat 53, a guide 55 is disposed outside and below the thermal decomposition tube 38 in parallel to the thermal decomposition tube 38, and a driving part 56 made of a magnet is movably disposed in the guide 55, whereby the supporting tool 54 is moved in the direction of the arrow X or Y in Fig. 8 by the magnetic force of the magnet driving part 56 from the outside of the thermal decomposition tube 38, instead of opening the lid 39a of the sample entrance 39 in moving the sample boat 53 having a sample S mounted thereon from the low temperature heating part 42 to the high temperature heating part 43. In a carbon differentiating and analyzing apparatus having this construction, the sample S can be moved in a state in which the outside air is shut off, without opening the lid 39a.

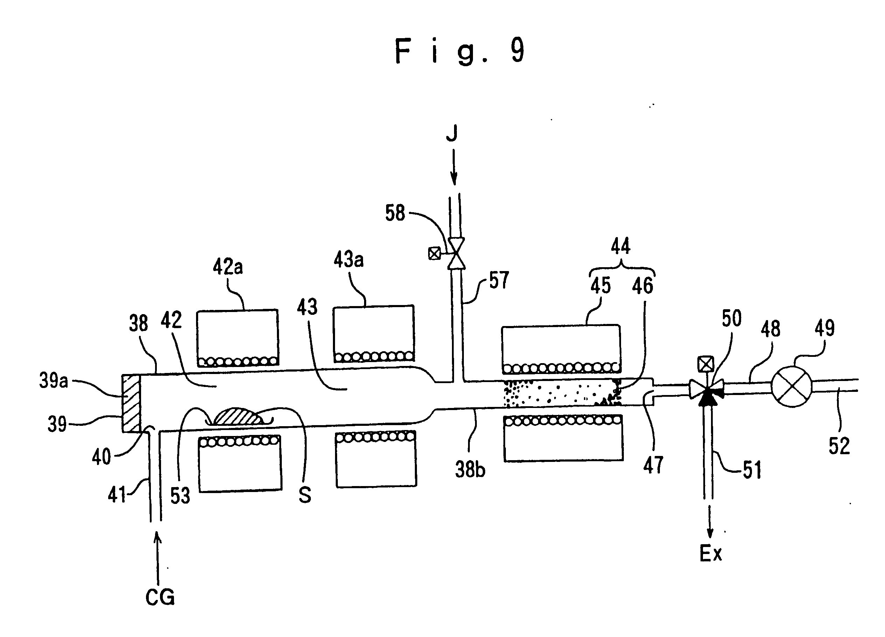

Further, referring to Fig. 9, a burning-aid gas introducing tube 57 for introducing a burning-aid gas J such as oxygen or air is connected at a site between the high temperature heating part 43 and the high temperature oxidizing part 44 of the thermal decomposition tube 38, whereby the burning-aid gas J is introduced into the thermal decomposition tube 38. In this case, the sample S can be burnt (oxidized) more smoothly and completely to generate CO₂ as desired. Here, the reference numeral 58 denotes a two-way solenoid valve as an on-off valve disposed midway in the burning-aid gas introducing tube 57.

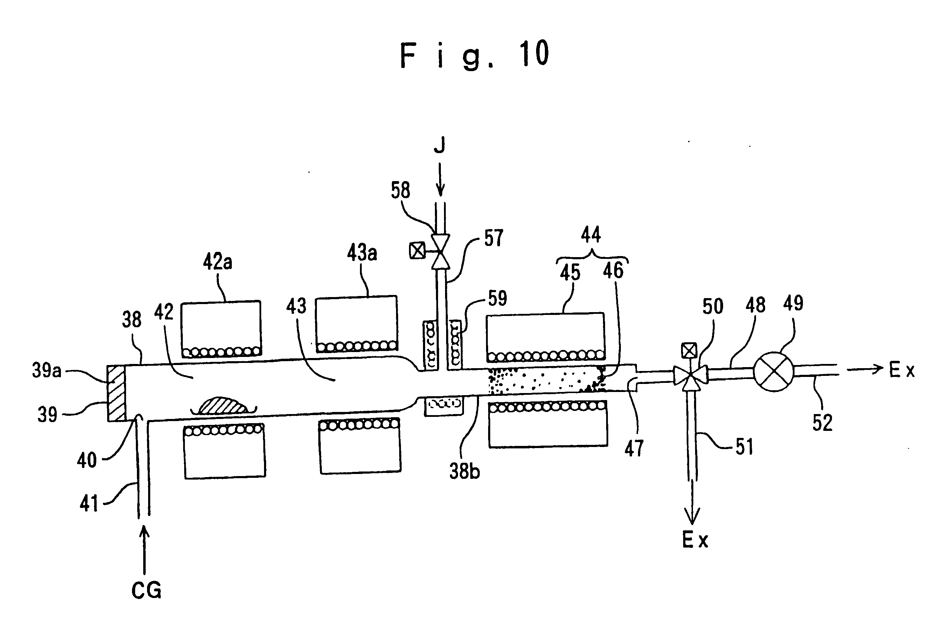

Further, referring to Fig. 10, a heater 59 may be disposed in the neighborhood of the connecting part of the burning-aid gas introducing tube 57 and the thermal decomposition tube 38, namely, in a part of the burning-aid gas introducing tube 57 near the thermal decomposition tube 38 and in the connecting part to the thermal decomposition tube 38, whereby the burning-aid gas J to be introduced into the thermal decomposition tube 38 may be heated in advance. In this case, the gas generated in the low temperature heating part 42 or in the high temperature heating part 43 will not be cooled by addition of the burning-aid gas J, so that CO₂ can be generated as desired.

As described above, according to the present invention, the organic carbon and the elemental carbon in a sample can be differentiated and analyzed. Further, the low temperature heating part and the high temperature heating part can be simultaneously operated and set at respective predetermined temperatures. This eliminates the need for the time required in raising the temperature or cooling in the carbon differentiation and analysis, thereby all the more reducing the time needed in the analysis.

Further, according to the apparatus of an embodiment HC and CO can be prevented from flowing to the CO_{2,} analyzer as they are, thereby improving the analysis precision of CO₂.

Also, according to the apparatus, low temperature CO in the presence of oxygen can be completely converted into CO₂, thereby preventing errors of the CO₂ analyzer caused by the generation of CO.

Thus, according to the apparatus of the present invention, the carbon differentiation and analysis can be carried out more speedily and more accurately.

## Claims

1. A method for analyzing particulate matter contained in gas,
comprising:
- providing a filter (2), which has captured particulate matter contained in engine emissions, in a heating furnace (1)
- firstly heating the filter to a predetermined temperature while feeding an inert gas into the heating furnace (1) to evaporate hydrocarbon and sulfate contained in the particulate matter into a generated gas,
- feeding a burning-aid gas such as O₂ to the generated gas for oxidation of the evaporated hydrocarbon,
- oxidizing the evaporated hydrocarbon into CO₂, and reducing the evaporated sulfate into SO₂,
- analyzing the CO₂ and the SO₂ with a gas analyzer unit (15), and
- thereafter heating the filter (2) while feeding oxygen into the heating furnace (1) to oxidize the particulate matter remaining on said filter (2) to convert it to CO₂, and
- analyzing the CO₂ with the gas analyzer unit (15).

2. The method according to claim 1,
wherein during heating of the filter (2) while feeding an inert gas into the heating furnace (1), firstly, low temperature heating is effected to an extent that the hydrocarbon of low boiling temperature should be evaporated, and thereafter, high temperature heating is effected to an extent that the hydrocarbon of high boiling temperature should be evaporated.

3. The method according to claim 1 or 2, wherein the sulfate is reduced using a reducing catalyst.

4. The method according to claims 1 or 2, wherein the sulfate is passed through heated quartz fibers.

5. The method according to any of the preceding claims, wherein the gas generated in the heating furnace (1) is passed through a gas flow passage and the downstream side of the gas flow passage is branched into two parallel gas flow passages (12, 13) and the evaporated hydrocarbon as well as the carbon dioxide are analyzed in a first analyzer (15A) and the sulfate is analyzed in a second analyzer (15B).

6. The method according to any of claims 1 to 4, wherein a first analyzer (15A) for analyzing evaporated hydrocarbon and carbon dioxide and a second analyzer (15B) for analyzing sulfate are disposed in series and the gas is sequentially passed through these analyzers.

7. The method according to claim 6, wherein before passing through the first analyzer (15A) and the second analyzer (15B) the gas is fed to an oxidation reduction processor (14) including an oxidation reduction catalyst.

8. The method according to any of the preceding claims, wherein the CO₂ is analyzed using an infrared spectrometer or a non-dispersive type infrared analyzer.

9. An apparatus for analyzing particulate matter contained in gas,
comprising:
- a gas feeder (4) configured to selectively feed an inert gas or oxygen to a heating furnace (1);
- a heating furnace (1) configured to heat a filter (2), which has captured the particulate matter contained in an engine emission to a predetermined temperature while inert gas or oxygen is fed to the heating furnace (1) to evaporate hydrocarbon and sulfate contained in the particulate matter;
- an oxidation (14A) and reduction (14B) processor configured to oxidize the evaporated hydrocarbon into CO₂ or to reduce the evaporated sulfate into SO₂, both generated during heating of the filter (2);
- a burning-aid gas introducing tube (57) for introducing a burning-aid gas such as O₂, the burning-aid gas introducing tube (57) being disposed between the heating furnace (1) and the oxidation processor (14A); and
- a gas analyzer unit (15) configured to determine the concentrations of CO₂ and SO₂ in the gas supplied from the oxidation (14A) and reduction (14B) processor.

## Patentansprüche

1. Verfahren zur Analyse von Teilchenmaterial, das in einem Gas enthalten ist, umfassend:
- Bereitstellen eines Filters (2), der eingefangenes Teilchenmaterial aufweist, das in Maschinenemissionen enthalten ist, in einem Wärmeofen (1),
- zunächst Erwärmen des Filters auf eine vorbestimmte Temperatur während ein inertes Gas in den Wärmeofen (1) eingeleitet wird, um Kohlenwasserstoff und Sulfat, die in dem Teilchenmaterial enthalten sind, in ein generiertes Gas freizusetzen,
- Zuführen eines Brennhilfsmittelgases, wie O₂, zu dem generierten Gas zur Oxidation des freigesetzten Kohlenwasserstoffs,
- Oxidieren des freigesetzten Kohlenwasserstoffs zu CO₂ und Reduzieren des freigesetzten Sulfats zu SO₂,
- Analysieren des CO₂ und des SO₂ mit einer Gasanalyseeinheit (15) und
- danach Erwärmen des Filters (2), während Sauerstoff in den Wärmeofen (1) zugeführt wird, um das auf dem Filter (2) verbleibende Teilchenmaterial zu oxidieren, um es in CO₂ umzuwandeln und
- Analysieren des CO₂ mit der Gasanalyseeinheit (15).

2. Verfahren nach Anspruch 1, wobei während des Erwärmens des Filters (2), während ein inertes Gas in den Wärmeofen (1) zugeführt wird, zunächst eine Niedertemperaturerwärmung in einem solchen Ausmaß durchgeführt wird, dass der Kohlenwasserstoff mit niedrigem Siedepunkt freigesetzt werden sollte, und danach eine Hochtemperaturerwärmung in einem Ausmaß durchgeführt wird, dass der Kohlenwasserstoff mit hohem Siedepunkt freigesetzt werden sollte.

3. Verfahren nach Anspruch 1 oder 2, wobei das Sulfat unter Verwendung eines Reduktionskatalysators reduziert wird.

4. Verfahren nach den Ansprüchen 1 oder 2, wobei das Sulfat über erwärmte Quarzfasern geführt wird.

5. Verfahren nach einem der vorhergehenden Ansprüche, wobei das Gas, das in den Wärmeofen (1) generiert wird, durch einen Gasflussdurchlass geführt wird und die nachgeschaltete Seite des Gasflussdurchlasses in zwei parallele Gasflussdurchlässe (12, 13) verzweigt wird und der freigesetzte Kohlenwasserstoff sowie das Kohlendioxid in einem ersten Analysator (15A) und das Sulfat in einem zweiten Analysator (15B) analysiert wird.

6. Verfahren nach einem der Ansprüche 1 bis 4, wobei ein erster Analysator (15A) für die Analyse des freigesetzten Kohlenwasserstoffs und Kohlendioxids und ein zweiter Analysator (15B) zur Analyse des Sulfats in Serie angeordnet sind und das Gas sequentiell durch diese Analysatoren geführt wird.

7. Verfahren nach Anspruch 6, wobei, bevor es durch den ersten Analysator (15A) und den zweiten Analysator (15B) geführt wird, das Gas zu einer Oxidations-Reduktions-Verarbeitungseinheit (14) geführt wird, die einen Oxidations-Reduktions-Katalysator umfasst.

8. Verfahren nach einem der vorhergehenden Ansprüche, wobei das CO₂ unter Verwendung eines Infrarotspektrometers oder eines Infrarotanalysators vom nicht-dispersiven Typ analysiert wird.

9. Vorrichtung zur Analyse von Teilchenmaterial, das in Gas enthalten ist, umfassend:
- eine Gaszuführvorrichtung (4), die so konfiguriert ist, um ein inertes Gas oder Sauerstoff zu einem Wärmeofen (1) selektiv zuzuführen;
- einen Wärmeofen (1), der so konfiguriert ist, um einen Filter (2), der Teilchenmaterial, das in einer Maschinenemission enthalten ist, eingefangen hat, auf eine vorbestimmte Temperatur zu erwärmen, während ein inertes Gas oder Sauerstoff dem Wärmeofen (1) zugeführt wird, um Kohlenwasserstoff und Sulfat, die in dem Teilchenmaterial enthalten sind, freizusetzen;
- eine Oxidations-(14A)- und Reduktions-(14B)-Verarbeitungseinheit, die so konfiguriert ist, um den freigesetzten Kohlenwasserstoff in CO₂ zu oxidieren oder das freigesetzte Sulfat in SO₂ zu reduzieren, wobei beide während des Erwärmens des Filters (2) generiert wurden;
- ein Brennhilfsgaseinführungsrohr (57) zum Einführen eines Brennhilfsgases, wie O₂, wobei das Brennhilfsgaseinführungsrohr (57) zwischen dem Wärmeofen (1) und der Oxidationsverarbeitungseinheit (14A) vorgesehen ist; und
- eine Gasanalyseeinheit (15), die so konfiguriert ist, um die Konzentrationen von CO₂ und SO₂ in dem Gas zu bestimmen, das von der Oxidations-(14A)- und Reduktions-(14B)-Verarbeitungseinheit zugeführt wird.

## Revendications

1. Procédé pour analyser une matière particulaire contenue dans un gaz, comprenant les étapes consistant à :
- agencer un filtre (2), lequel a capturé de la matière particulaire contenue dans des émissions de moteur, dans un four de chauffage (1),
- chauffer d'abord le filtre jusqu'à une température prédéterminée tout en alimentant un gaz inerte dans le four de chauffage (1) afin d'évaporer les hydrocarbures et les sulfates contenus dans la matière particulaire dans un gaz généré,
- transférer un gaz d'aide à la combustion tel que O₂ vers le gaz généré en vue d'une oxydation des hydrocarbures évaporés,
- oxyder les hydrocarbures évaporés en CO₂, et réduire les sulfates évaporés en SO₂,
- analyser le CO₂ et le SO₂ à l'aide d'une unité d'analyse de gaz (15), et
- chauffer ensuite le filtre (2) tout en transférant de l'oxygène dans le four de chauffage (1) afin d'oxyder la matière particulaire subsistant sur ledit filtre (2) pour la convertir en CO₂, et
- analyser le CO₂ avec l'unité d'analyse de gaz (15).

2. Procédé selon la revendication 1,
dans lequel, pendant le chauffage du filtre (2) tout en transférant un gaz inerte dans le four de chauffage (1), un chauffage à basse température est d'abord effectué jusqu'à un point où les hydrocarbures à basse température d'ébullition devraient être évaporés, et un chauffage à haute température est ensuite effectué jusqu'à un point où les hydrocarbures à haute température d'ébullition devraient être évaporés.

3. Procédé selon la revendication 1 ou 2, dans lequel les sulfates sont réduits en utilisant un catalyseur de réduction.

4. Procédé selon les revendications 1 ou 2, dans lequel les sulfates passent à travers des fibres de quartz chauffées.

5. Procédé selon l'une quelconque des revendications précédentes, dans lequel le gaz généré dans le four de chauffage (1) passe à travers un canal d'écoulement de gaz et le côté aval du canal d'écoulement de gaz se divise en deux canaux d'écoulement de gaz parallèles (12, 13) et les hydrocarbures évaporés ainsi que le dioxyde de carbone sont analysés dans un premier analyseur (15A) et les sulfates sont analysés dans un second analyseur (15B).

6. Procédé selon l'une quelconque des revendications 1 à 4, dans lequel un premier analyseur (15A) destiné à analyser les hydrocarbures évaporés et le dioxyde de carbone et un second analyseur (15B) destiné à analyser les sulfates sont disposés en série, et le gaz passe séquentiellement à travers ces analyseurs.

7. Procédé selon la revendication 6, dans lequel, avant de passer par le premier analyseur (15A) et le second analyseur (15B), le gaz est transféré vers un dispositif de traitement par oxydoréduction (14) incluant un catalyseur d'oxydoréduction.

8. Procédé selon l'une quelconque des revendications précédentes, dans lequel le CO₂ est analysé en utilisant un spectromètre infrarouge ou un analyseur infrarouge de type non dispersif.

9. Appareil destiné à analyser une matière particulaire contenue dans un gaz, comprenant :
- un dispositif d'alimentation en gaz (4) configuré pour alimenter sélectivement un gaz inerte ou de l'oxygène vers un four de chauffage (1) ;
- un four de chauffage (1) configuré pour chauffer un filtre (2), lequel a capturé la matière particulaire contenue dans une émission de moteur jusqu'à une température prédéterminée alors que le gaz inerte ou l'oxygène est transféré vers le four de chauffage (1) pour faire évaporer les hydrocarbures et les sulfates contenus dans la matière particulaire ;
- un dispositif de traitement par oxydation (14A) et réduction (14B) configuré pour oxyder les hydrocarbures évaporés en CO₂ ou pour réduire les sulfates évaporés en SO₂, tous deux générés pendant le chauffage du filtre (2) ;
- un tube (57) d'introduction de gaz d'aide à la combustion destiné à introduire un gaz d'aide à la combustion tel que O₂, le tube (57) d'introduction de gaz d'aide à la combustion étant disposé entre le four de chauffage (1) et le dispositif de traitement par oxydation (14A) ; et
- une unité d'analyse de gaz (15) configurée pour déterminer les concentrations de CO₂ et de SO₂ dans le gaz fourni à partir du dispositif de traitement par oxydation (14A) et réduction (14B).
